# EUROPEAN PATENT SPECIFICATION

(11) **EP 2 961 458 B1**
(45) Date of publication and mention of the grant of the patent: **10.01.2018**
(21) Application number: 14708448.7
(22) Date of filing: 21.02.2014
(51) Int. Cl.: A61M 25/04, A61M 25/00, A61M 25/06

(54) **GUIDE EXTENSION CATHETER WITH A RETRACTABLE WIRE**
FÜHRUNGSERWEITERUNGSKATHETER MIT EINEM ZURÜCKZIEHBAREN DRAHT
CATHÉTER D'EXTENSION DE GUIDAGE

(30) Priority: 01.03.2013 US 201361771722 P
(43) Date of publication of application: 06.01.2016
(73) Proprietor: Boston Scientific Scimed, Inc., Maple Grove, MN 55311 (US)
(72) Inventor: WANG, Huisun, Maple Grove, Minnesota 55311 (US); ANDERSON, James M., Corcoran, MN 55357 (US); PLESSEL, Justin E., Maple Grove, Minnesota 55311 (US)
(74) Representative: Vossius & Partner Patentanwälte Rechtsanwälte mbB
(86) International application number: PCT/US2014/017728
(87) International publication number: WO 2014/133897

(56) References cited:
- EP-A1- 1 639 951
- US-A1- 2004 059 179
- US-A1- 2009 264 865
- US-A1- 2010 234 876
- US-B1- 6 537 294

## Description

### Technical Field

The present disclosure pertains to medical devices and methods for manufacturing the same. More particularly, the present disclosure pertains to elongated intracorporeal medical devices including a guide extension catheter having a retractable wire.

### Background

A wide variety of intracorporeal medical devices have been developed for medical use, for example, intravascular use. Some of these devices include guidewires, catheters, and the like. These devices are manufactured by any one of a variety of different manufacturing methods and may be used according to any one of a variety of methods. US 2004/0059179 A1 relates to a system and method for locally delivering fluids or agents into branch blood vessels or body lumens from a main vessel or lumen, respectively, and in particular into renal arteries extending from an aorta in a patient. EP 1639 951 A1 discloses an intravascular foreign matter suction assembly for sucking a foreign matter existing in a blood vessel and comprising a guiding catheter and a suction catheter. The suction catheter comprises a tubular portion provided on a distal side of the suction catheter, the tubular portion including a distal tube end and a proximal tube end and a solid wire-like portion provided at the proximal tube end. Of the known medical devices and methods, each has certain advantages and disadvantages. There is an ongoing need to provide alternative medical devices as well as alternative methods for manufacturing and using medical devices.
US 2009/264865 A1 discloses an insertion assisting tool including a distal end portion having a distally closed core member insertion hole for a core member.

### Brief Summary

In accordance with the present invention, there is provided a guide extension catheter as defined in claim 1 and a medical extension assembly as defined in claim 9.

In addition, further advantageous embodiments follow from the dependent claims.

A method of accessing a coronary artery is described. The method may include providing a guide catheter, advancing the guide catheter through a first outer diameter blood vessel to a location adjacent to an ostium of a coronary artery, providing a guide extension catheter, and advancing a treatment catheter through the guidewire. The guide extension catheter may include a proximal shaft having a first outer diameter, and a distal sheath attached to the proximal shaft and having a second outer diameter greater than the first outer diameter. The distal sheath has a central lumen defined therein and a secondary lumen formed along a wall surface of the distal sheath. The guide extension catheter may be advanced through the guide catheter to a position where at least a portion of the distal sheath extends distally beyond a distal end of the guide catheter and into the coronary artery, and advancing a treatment catheter through the guide catheter.

The above summary of some embodiments is not intended to describe each disclosed embodiment or every implementation of the present disclosure. The Figures, and Detailed Description, which follow, more particularly exemplify these embodiments.

### Brief Description of the Drawings

The disclosure may be more completely understood in consideration of the following detailed description in connection with the accompanying drawings, in which:
Figure 1 is a plan view illustrating an example guide catheter advanced through the aorta to the ostium of a coronary artery;
Figure 2 is a plan view illustrating an example guide extension catheter used in conjunction with a guide catheter;
Figure 3 is a cross-sectional side view of an example guide extension catheter;
Figure 4 is a cross-sectional side view of the example guide extension catheter and an example guide catheter;
Figure 5 is a side view of an exemplary guide extension catheter, according to the present disclosure;
Figure 6 is a side view of the guide extension catheter of Figure 5 with a retractable wire, according to the present disclosure;
Figure 7 is a side view of a guide extension catheter with the retractable wire in an extended position into a secondary lumen, according to the present disclosure;
Figure 8 is a side view of an exemplary guide extension catheter including the retractable wire, according to the present disclosure;
Figure 9 is a cross-sectional view of a proximal shaft with the retractable wire taken on a plane 9-9 of Figures 7-8;
Figure 10 is a cross-sectional view of a distal sheath with the retractable wire disposed in the secondary lumen taken on a plane 10-10 of Figures 7-8;
Figure 11 is a cross-sectional view of the distal sheath with the secondary lumen in an open configuration taken on a plane 9-9;
Figure 12 is a cross-sectional view of an example distal sheath with a secondary lumen in a first configuration; and
Figure 13 is a cross-sectional view of an example distal sheath with a secondary lumen in a second configuration.

While the disclosure is amenable to various modifications and alternative forms, specifics thereof have been shown by way of example in the drawings and will be described in detail. It should be understood, however, that the intention is not to limit the invention to the particular embodiments described. On the contrary, the intention is to cover all modifications, equivalents, and alternatives falling within the scope of the disclosure as defined by the appended claims.

### Detailed Description

For the following defined terms, these definitions shall be applied, unless a different definition is given in the claims or elsewhere in this specification.

All numeric values are herein assumed to be modified by the term "about," whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

It is noted that references in the specification to "an embodiment", "some embodiments", "other embodiments", etc., indicate that the embodiment described may include one or more particular features, structures, and/or characteristics. However, such recitations do not necessarily mean that all embodiments include the particular features, structures, and/or characteristics. Additionally, when particular features, structures, and/or characteristics are described in connection with one embodiment, it should be understood that such features, structures, and/or characteristics may also be used connection with other embodiments whether or not explicitly described unless clearly stated to the contrary.

The following detailed description should be read with reference to the drawings in which similar elements in different drawings are numbered the same. The drawings, which are not necessarily to scale, depict illustrative embodiments and are not intended to limit the scope of the invention.

Embodiments of the present disclosure may include a medical device for the delivery of diagnostic or therapeutic modalities. The medical device may take the form of a guide extension catheter having a proximal shaft, a distal sheath attached to the proximal shaft. The guide catheter may further include a retractable wire. The retractable wire can be used to help a tractable tip of the guide extension catheter to cross a lesion within a blood vessel of a patient's body. The retractable wire can be retracted back to the proximal shaft after the tractable tip passed the lesion. The retractable wire can help to cross the challenged lesions within the patient's body. Further, the distal sheath may include a collapsible secondary lumen. The secondary lumen may collapse when the tractable wire has been retracted back to the proximal shaft.

Many of the following examples illustrate implementations in which the catheter may be employed to navigate through a constricted portion of a coronary artery. It will be understood that this choice is merely exemplary and the catheter shaft may be used in any desired body location requiring diagnostic or therapeutic modalities without departing from the scope of the present disclosure.

For purposes of this disclosure, "proximal" refers to the end closer to the device operator during use, and "distal" refers to the end further from the device operator during use.

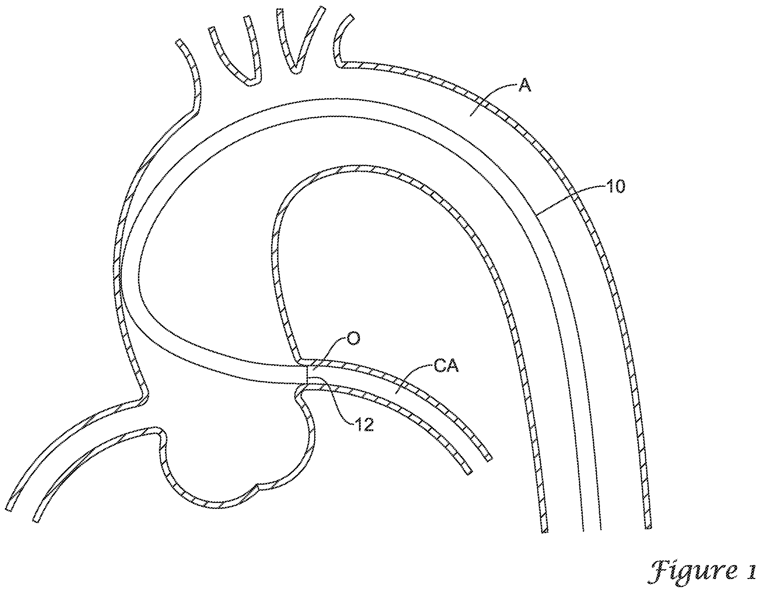

Minimally-invasive cardiac interventions such as percutaneous transluminal coronary angioplasty are widely utilized throughout the world. These procedures may include the use of a guide catheter. For example, a guide catheter 10 may be advanced through a blood vessel such as the aorta A to a position adjacent to the ostium O of a (e.g., left and/or right) coronary artery CA as illustrated in Figure 1. When so positioned, a treatment catheter (e.g., balloon catheter, stent delivery system, etc.) may be advanced through guide catheter 10 and into the coronary artery CA to a target location where the treatment catheter may be used to perform the appropriate cardiac intervention.

In order for the treatment catheter to efficiently reach the intended target location, maintaining the position of guide catheter 10 at the ostium O of the coronary artery CA may be desirable. For example, given that the heart may be beating during the intervention (and/or other factors), the guide catheter 10 may lose its positioning or otherwise be shifted so that it no longer is positioned to efficiently guide the treatment catheter to the coronary arteries. Thus, the distal end 12 of guide catheter 10 may be shifted away from the ostium O of the coronary artery CA. Because of the shift away from the ostium O, access to the coronary arteries CA may require repositioning of guide catheter 10 in order to bring the distal end 12 back into engagement with the ostium O of the coronary artery CA.

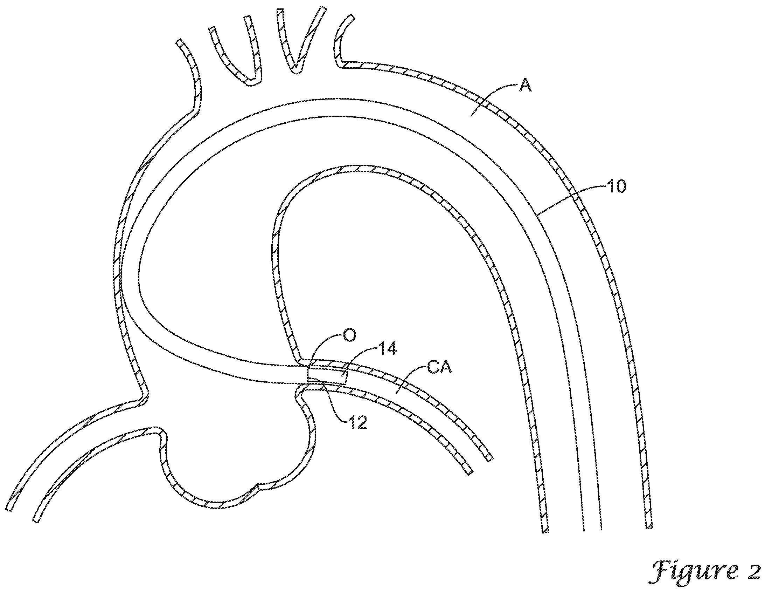

Disclosed herein are medical devices and described herein are methods for making and using medical devices that may improve access to the coronary arteries CA. For example, Figure 2 illustrates a guide extension catheter 14 extending through guide catheter 10 and beyond distal end 12 of guide catheter 10 into the coronary artery CA. Because, for example, guide extension catheter 14 may extend beyond distal end 12 of guide catheter 10, guide extension catheter 14 may extend beyond the ostium O of the coronary artery CA and into a portion of the coronary artery CA. By extending beyond the ostium O, the extension catheter 14 may stabilize the positioning of guide catheter 10 and allow for improved access to the coronary artery CA for a number of cardiac interventions.

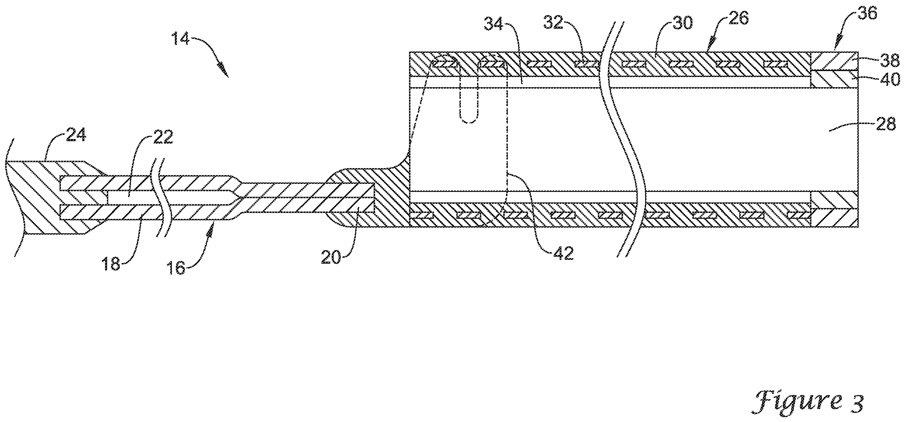

Figure 3 is a cross-sectional side view of guide extension catheter 14. Here it can be seen that guide extension catheter 14 may include a proximal shaft or member 16. Proximal member 16 may include a proximal portion 18 and a distal or ribbon portion 20. Proximal portion 18 may have a lumen 22 defined therein. In some embodiments, lumen 22 extends along the entire length of proximal portion 18. In other embodiments, lumen 22 extends along only a portion of the length of proximal portion 18. In addition, proximal portion 18 may include both proximal and distal openings (e.g., positioned at the proximal and distal end of proximal portion 18) such that lumen 22 is "open" on both ends. Alternatively, one or both of the ends of proximal portion 18 may be closed or otherwise sealed. For example, the distal end of proximal portion 18 may be closed. In some of these and in other embodiments, proximal portion 18 may have an opening or port (not shown) formed in the wall of proximal portion 18 and spaced from the proximal and/or distal end of proximal portion 18. The port may or may not be in fluid communication with lumen 22. A hub 24 may be attached to proximal portion 18.

A distal sheath 26 may be attached to proximal member 16. The distal sheath 26 may have a lumen 28 formed therein. In general, lumen 28 (and/or the inner diameter of distal sheath 26) may be larger than lumen 22 (and/or the inner diameter of proximal portion 18) and may be larger than the outer diameter of proximal member 16. Accordingly, lumen 28 may be sufficiently large so as to allow a therapeutic catheter (e.g., balloon catheter, stent delivery system, etc.) to pass there through. For example, when guide extension catheter 14 is positioned within guide catheter 10, the therapeutic catheter may extend within guide catheter 10 alongside proximal member 16 and through lumen 28 of distal sheath 26.

The distal sheath 26 may include a body portion 30. In some embodiments, the body portion 30 is made from one or more polymers including any of those disclosed in this application. This may include the use of polymers with a differing durometer along the length of body portion 30. For example, a more proximal section of body portion 30 may include a polymer with a higher durometer and a more distal section of body portion 30 may include a polymer with a lower durometer. Portions of all of the length of body portion may be loaded with or otherwise include a radiopaque material. Body portion 30 may also include a reinforcement member 32. The form of reinforcement member 32 may vary.

An inner liner or layer 34 may be disposed along an inner surface of body portion 30. The form of the inner liner 34 may vary. For example, the inner liner 34 may be a lubricious liner or otherwise include a lubricious material such as polytetrafluoroethylene. A tip member 36 may be attached to the body portion 30, for example at a distal end of the body portion 30. In some embodiments, the tip member 36 may be a single layer of material. Alternatively, the tip member 36 may include an outer layer 38 and an inner layer 40. The outer layer 38 and the inner layer 40 may be formed from the same material. In some of these embodiments, the outer layer 38 and the inner layer 40 may include the same polymeric material and each may be loaded with the same or different radiopaque materials. For example, the inner layer 40 may include a polyether block amide loaded with approximately 75-95% (e.g., about 90%) by weight tungsten and the outer layer 38 may include a polyether block amide loaded with approximately 30-50% (e.g., 40%) by weight bismuth subcarbonate. These are just examples. In other embodiments, the outer layer 38 and inner layer 40 may be made from different materials.

The distal sheath 26 may be attached to a ribbon portion 20 of the proximal member 16. The arrangement and/or configuration of the attachment between the ribbon portion 20 and the distal sheath 26 may vary. For example, the distal sheath 26 may have an opening or lumen formed in tube wall thereof and the ribbon portion 20 may be disposed within the opening. This may include necking, skiving, or pinching down the ribbon portion 20 and inserting the necked down portion into the opening. In some embodiments, inserting the ribbon portion 20 into the opening may secure the proximal member 16 to the distal sheath 26 via a mechanical bond. In some of these and in other embodiments, additional and/or alternative bonding may be utilized including those bonding mechanisms commonly used for medical devices (e.g., adhesive bonding, welding, thermal bonding, brazing, etc.). Other attachment mechanisms are also contemplated for attaching the proximal member 16 to the distal sheath 26 including direct bonding (e.g., adhesive bonding, thermal bonding, welding, brazing, etc.), bonding that is facilitated by a third component such as a metal or polymer collar 42 that may be bonded between the ribbon portion 20 and the distal sheath 26.

The guide extension catheter 14 may also include a number of coatings that may, for example, reduce friction. For example, the proximal member 16 may have an inner and/or outer coating that includes a hydrophilic polymer that may reduce friction during tracking. An example coating may include BAYER CL-100, BIOSLIDE, NG-HPC, SLIP COAT, MDX, or the like. These are just examples. Other materials are contemplated including those disclosed herein.

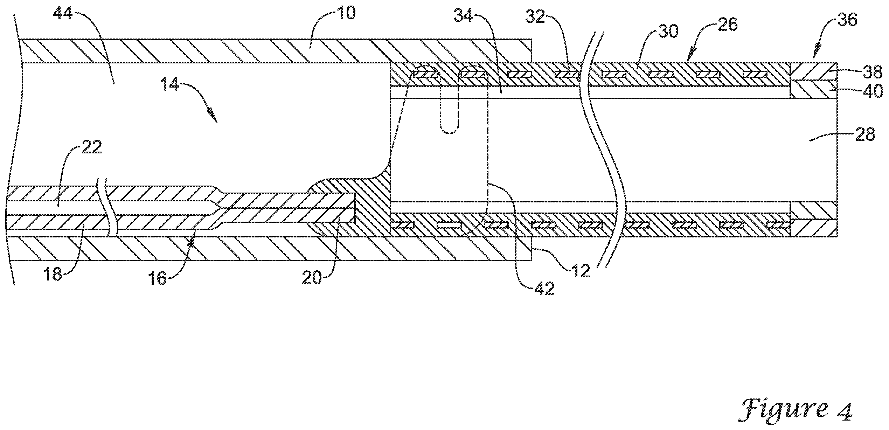

Figure 4 illustrates the guide extension catheter 14 disposed within guide catheter 10 (e.g., disposed within a lumen 44 defined within guide catheter 10). As shown, the distal sheath 26 may be arranged to extend distally out from the distal end 12 of the guide catheter 10. When so arranged, the distal sheath 26 may engage the ostium O and/or extend within a portion of the coronary artery CA (see Figure 1) to help maintain the position of the guide catheter 10 and improve access to the coronary artery CA. The proximal member 16 may be designed to be sufficiently small (while still being sufficiently sized and configured for pushability) so as to take up relatively little space within the interior or the lumen 44 of the guide catheter 10. Accordingly, the use of the guide extension catheter 14 allows for a therapeutic catheter or medical device to be advanced through the guide catheter 10 in order to reach the desired target location for the intervention. In some embodiments, the proximal member 16 may contact the inner wall surface of the guide catheter 10, which may provide even more space.

When designing guide extension catheter 14, it may be desirable to incorporate structures that may provide structural "push" support. However, the use of such structures may add bulk and/or stiffness to the guide extension catheter 14. Disclosed herein are guide extension catheters that include pushing structures and/or stiffening members.

The stiffening members may be removable from the guide extension catheter 14 such that additional push support and/or stiffness can be added/removed as needed during an intervention. The use of a removable stiffening member may provide additional space within the guide catheter for other therapeutic devices to pass therethrough, upon removal of the stiffening member.

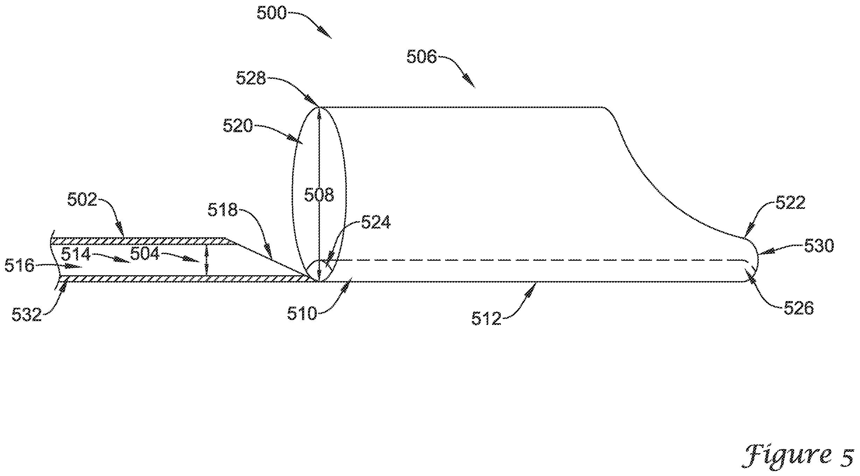

Figure 5 is a side view schematic of an exemplary guide extension catheter 500, according to the present disclosure. The guide extension catheter 500 may include a proximal shaft 502 and a distal sheath 506. It can be appreciated that the form and/or structural configuration of proximal shaft 502 and/or distal sheath 506 may resemble other proximal shafts and distal sheaths disclosed herein. Further, the proximal shaft 502 may be formed using a biocompatible material having elasticity properties. The proximal shaft 502 may be an elongate member coupled distally to the distal sheath 506 via appropriate coupling mechanism including any of those disclosed herein. Alternatively the proximal shaft 502 may be a hypotube-like structure having a proximal end 532, and a tubular member 514 with an open distal end 518. The coupling mechanism may include, but are not limited to, necking, skiving, or pinching down a ribbon portion (not shown), a collar, or the like. In some of these and in other embodiments, additional and/or alternative bonding may be utilized including those bonding mechanisms commonly used for medical devices (e.g., adhesive bonding, welding, thermal bonding, brazing, etc.). Other attachment/coupling mechanisms are also contemplated for attaching the proximal shaft 502 to the distal sheath 506 including direct bonding (e.g., adhesive bonding, thermal bonding, welding, brazing, etc.), bonding that is facilitated by a third component such as a metal or polymer collar that may be bonded between the ribbon portion and the distal sheath 506.

Further, the tubular member 514 of the proximal shaft 502 may define a lumen 516. The lumen 516 may have a constant diameter or the lumen 516 may have a changing or tapered diameter in some embodiments. Further, the lumen 516 may extend entire length of the proximal shaft 502. In some embodiments, the lumen 516 may extend along only a portion of the length of the proximal shaft 502. The proximal shaft 502 may also have a first outer diameter 504. Moreover, the proximal shaft 502 may have any cross-sectional shape, such as cylindrical, circular, rhombic, rectangular, oval, semicircular, or the like, suitable for insertion into a guide catheter 10 as discussed above.

The distal sheath 506 may have a proximal end 528 and a distal end 522 with a central lumen 520 defined between those ends. The distal end 518 of the proximal shaft 502 may be attached to the proximal end 528 of the distal sheath 506. The proximal and distal ends 528, 522, may have a proximal opening and a distal opening, respectively and these openings may define the central lumen 520. The central lumen 520 of the distal sheath 506 may be larger than the lumen 516 of the proximal shaft 502. In particular, the central lumen 520 may be sufficiently large to allow a therapeutic device, such as a balloon catheter or stent delivery system, for example, to be carried through the central lumen 520. In other words, the central lumen 520 may be sized to accommodate a therapeutic device including those that are larger than a guidewire. Thus, when the guide extension catheter 500 is positioned within guide catheter 10, the therapeutic device may be disposed within central lumen 520. The distal sheath 506 may have a second outer diameter 508 greater than the first outer diameter 504. The distal sheath 506 may have an outer diameter 508 that approximates the inner diameter of a guide catheter 10 so that the guide extension catheter 500 may extend through the guide catheter 10 appropriately. The distal sheath 506 may have a uniform cross-section or diameter from its proximal end 528 to the distal end 522, or that cross-section and diameter may vary through its length.

A tapered distal tip 530 may be included at the distal end 522 of guide extension catheter 500, in the form of a tubular snout or projection extending distally from the guide extension catheter 500. The distal tip 530 may be formed integrally with or may attach to the distal sheath 506. The distal tip 530 may assist the guide extension catheter 500 to cross a lesion in a blood vessel within a patient's body. The distal tip 530 may include a radiopaque material in order to visualize the tip with the help of X-ray or other imaging technique..

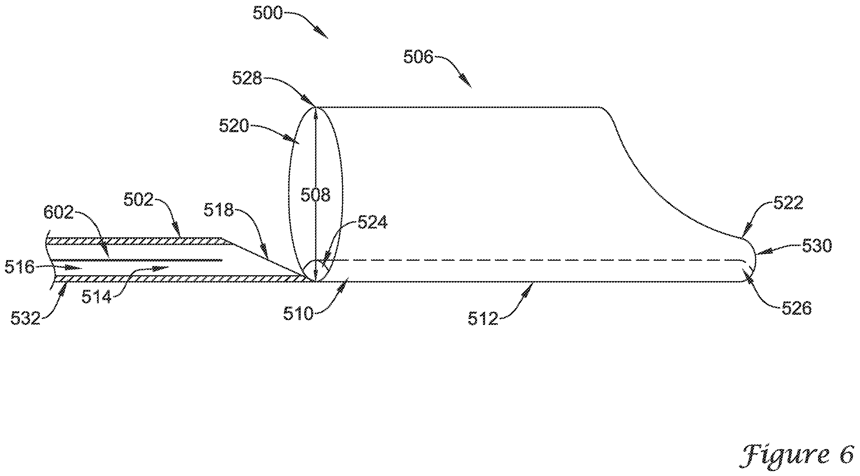

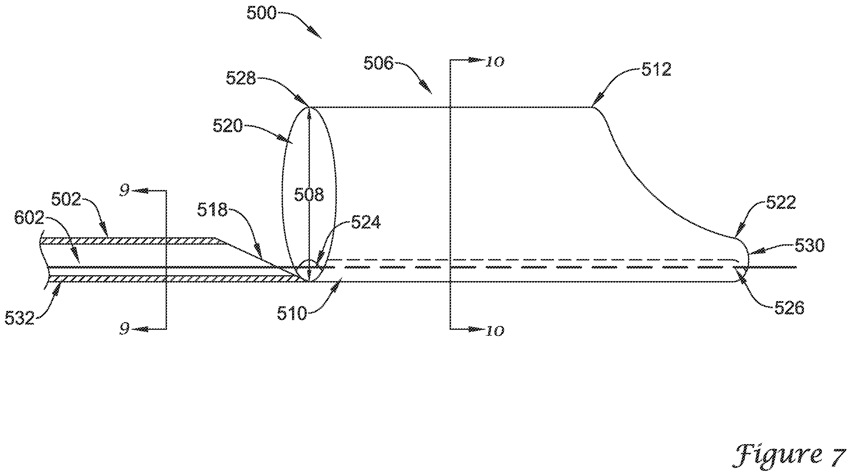

Further, the distal sheath 506 may have a secondary lumen 510 formed along a wall surface 512 (or within the wall of distal sheath 506) of the distal sheath 506. In some embodiments, the lumen 516 formed within the proximal shaft 502 may be longitudinally-aligned with the secondary lumen 510 such that, a retractable wire (See Figure 7) can pass through both the lumens i.e. 516 and 510. Further, the diameter of the secondary lumen 510 may be smaller than the diameter of the central lumen 520. A range of biocompatible polymers such as, but not limited to, polyurethane, silicone, and so forth, may be used for construction of the secondary lumen 510. The secondary lumen 510 may open into the central lumen 520 of the distal sheath 506 and may extend distally to the end of the distal tip 530. The distal tip 530 may have a smaller outer diameter than the distal sheath 506. Particularly, the secondary lumen 510 may have a third outer diameter that is smaller than the second outer diameter 508 of the distal sheath 506. In addition, the distal tip 530 may have a smaller inner diameter than the distal sheath 506, so that, for example, the diameter of the central lumen 520 approximates the outer diameter of the guidewire (See Figure 6). Further, the secondary lumen 510 has a proximal opening 524 towards the proximal end 528 of the distal sheath 506 and a distal opening 526 towards the distal end 522 of the distal sheath 506.

Further, the precise form of the distal sheath 506 may vary. For example, in one embodiment, the distal sheath 506 may include a supporting member in the form of braid, rod, coil, mesh or similar structure. The distal sheath 506 may also include radiopaque material, either as a radiopaque marker band, as a polymeric material doped with radiopaque material, or the like. As discussed above, the distal sheath 506 may attach mechanically to the proximal shaft 502. In other embodiments, bonding mechanism including adhesive bonding, thermal bonding, welding, brazing, for example may be used. Also, a collar (not shown) may be disposed at the distal end 518 of the proximal shaft 502 to attach the distal sheath 506 to the proximal shaft 502. Other attachment mechanisms are contemplated.

In some embodiments, the guide extension catheter 500 may include a guidewire or a retractable wire that may extend through a central lumen 520 of the distal sheath 506. A range of biocompatible polymers such as, but not limited to, polyurethane, silicone, and so forth, may be used for construction of the guide extension catheter 500. Silicone is one of the most common choices because it is inert and biocompatible.

Further, the guide extension catheter 500 may include a number of coatings that may, for example, reduce friction. For example, proximal shaft 502 may have an inner and/or outer coating that includes a hydrophilic polymer that may reduce friction during tracking. An example coating may include BAYER CL-100, BIOSLIDE, NG-HPC, SLIP COAT, MDX, or the like. These are just examples; other materials are contemplated including those disclosed herein.

Figure 6 is a side view of the exemplary guide extension catheter 500 with a retractable wire 602, according to the present disclosure. As shown, the guide extension catheter 500 may further include the retractable wire 602 disposed within at least a portion of the proximal shaft 502. In an embodiment, the retractable wire 602 may be disposed completely within the proximal shaft 502. The retractable wire 602 can help the distal tip 530 of the distal sheath 506 to cross one or more lesions in a blood vessel within the patient's body. The retractable wire 602 can be retracted back to the proximal shaft 502 after the distal tip 530 has passed or crossed the lesion. In some embodiments, the retractable wire 602 may be retracted only from the distal sheath 506. In alternate embodiments, the retractable wire 602 may be retracted completely from the guide extension catheter 500. The retractable wire 602 may be retracted by using suitable mechanisms at the proximal end 532 of the proximal shaft 502. In an embodiment, the retractable wire 602 is flexible. In some embodiments, the mechanism may include a button at the proximal end 532 of the proximal shaft 502. Further, the retractable wire 602 may be retracted or extended by pressing a button location at the proximal end 532 of the proximal shaft 502.

In some embodiments, the retractable wire 602 may be disposed within the proximal shaft 502 as well as in the secondary lumen 510 of the distal sheath 506 when the retractable wire 602 is extended. As shown in Figure 7, the retractable wire 602 can be disposed within the proximal shaft's lumen 516 and may extend to the secondary lumen 510 of the distal sheath 506.

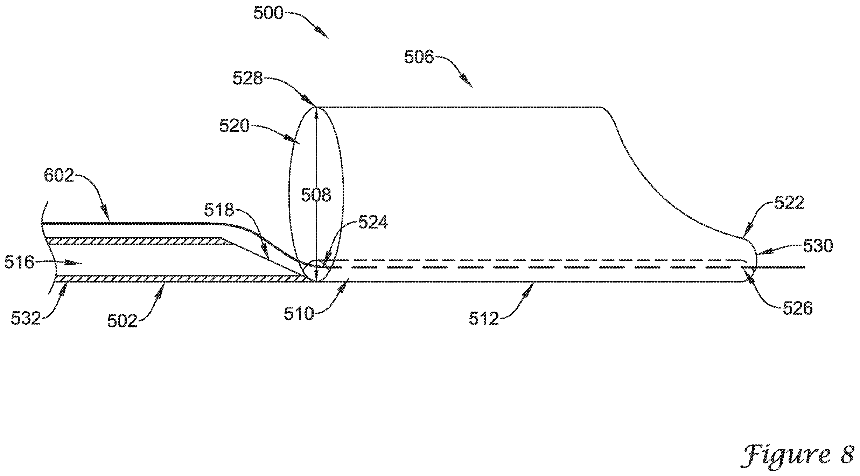

In an embodiment, the retractable wire 602 may be disposed along an exterior of the proximal shaft 502 and may extend into the secondary lumen 510 from the proximal opening 524. Figure 8 shows a side view of the guide extension catheter 500 of Figure 5 including the retractable wire 602 that may extend within the secondary lumen 510 from the proximal opening 524. Further as shown, the retractable wire 602 can extend distally from the distal end 522 of the distal sheath 506. For example, the retractable wire 602 may extend distally from the distal opening 526 at the distal tip 530. In this embodiment, the retractable wire 602 is not extended through the proximal shaft 502 and only passing through the secondary lumen 510.

The retractable wire 602 may help the distal sheath 506 or the guide extension catheter 500 to cross challenged sections by functioning as a "buddy wire." This may allow another diagnostic and/or treatment medical device to be advanced to an area of interest. In an embodiment, the width, length and/or density of the retractable wire 602 may be changed depending on application of the guide extension catheter 500 without need to change the guide catheter.

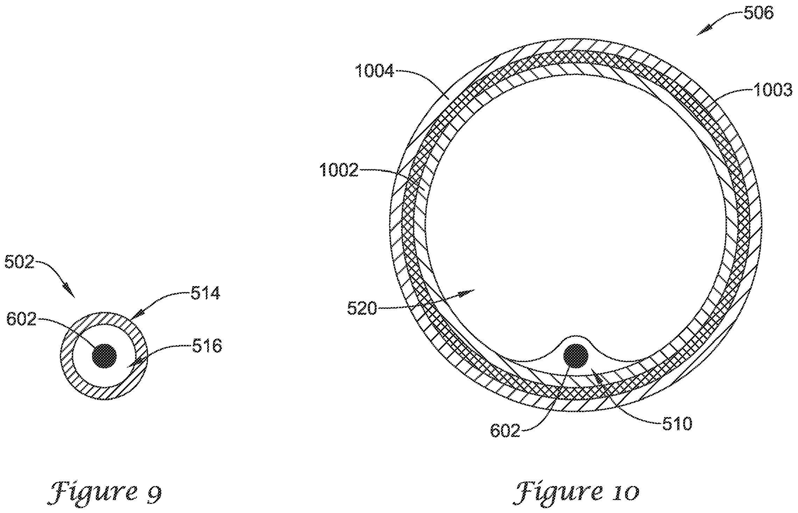

Figure 9 is a cross-sectional view of the proximal shaft 502 with the retractable wire 602 taken on a plane 9-9 of Figures 7-8. As shown, the retractable wire 602 can be disposed within the proximal shaft's lumen 516 of the tubular member 514. The retractable wire 602 can be formed using a suitable material such as, but not limited to, stainless steel, nickel-titanium alloy, and so forth.

Figure 10 is a cross-sectional view of the distal sheath 506 with the retractable wire 602 disposed in the secondary lumen 510 taken on a plane 10-10 of Figures 7-8. An outer surface of the distal sheath 506 may include a radiopaque material disposed thereon to visualize the distal sheath 506 or the guide extension catheter 500 with the help of X-ray or the like imaging technique. The distal sheath 506 may further include an inner layer 1002, an outer layer 1004, and a reinforcing member 1003 to provide strength and structural integrity to the distal sheath 506 or the guide extension catheter 500. For example, reinforcing member 1003 may be a braid, coil, mesh, or the like designed to maintain the structural integrity of the distal sheath 506.

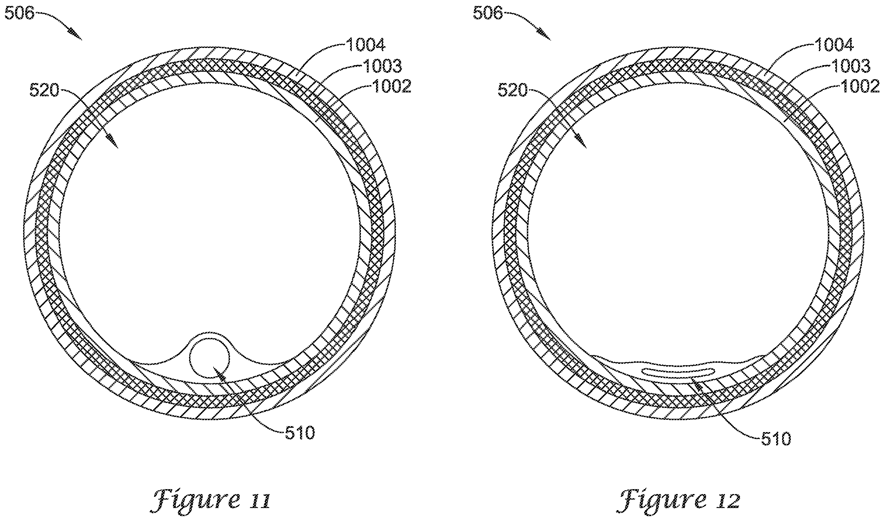

Further, the secondary lumen 510 may or may not be collapsible. In some embodiments, the secondary lumen 510 may be configured to shift between an open configuration and a collapsed configuration. Figure 11 is a cross-sectional view of the distal sheath 506 with the secondary lumen 510 in an open configuration taken on a plane 9-9 of Figures 7-8. The secondary lumen 510 is in the open or expanded configuration when the retractable wire 602 is disposed within. In some embodiments, the secondary lumen 510 does not collapse, e.g., it maintains its shape even when the retractable wire 602 is retracted therefrom.

Figure 12 is a cross-sectional view of the distal sheath 506 in a closed or collapsed configuration taken on a plane 10-10 of Figures. 7-8. As shown, the secondary lumen 510 may collapse when the retractable wire 602 is retracted from the secondary lumen 510 (or the distal sheath 506). The retractable wire 602 can be retracted back to the proximal shaft 502 after the distal tip 530 has passed the lesion(s) in the blood vessel. The secondary lumen 510 may collapse to maximize the inner pass-way (or the central lumen 520) within the distal sheath 506 of the guide extension catheter 500. The maximized inner pass-way (or the central lumen 520) can allow various therapeutic devices or treatment catheters to pass easily through the distal sheath 506.

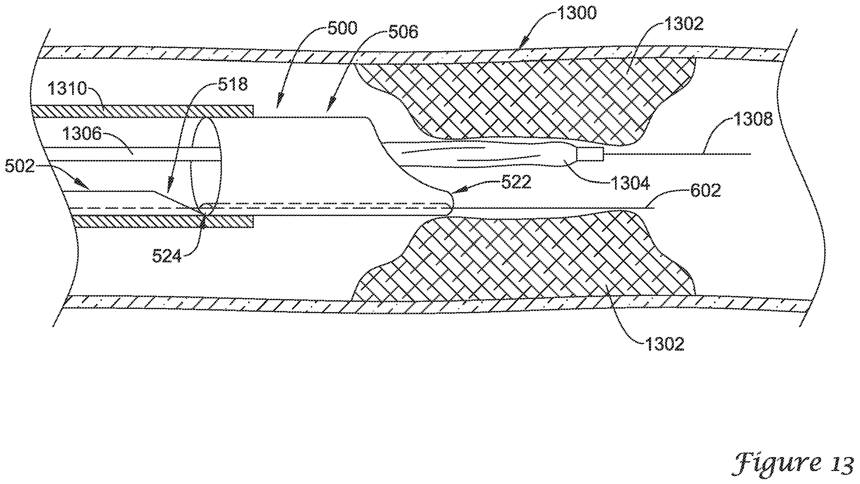

Figure 13 is a side view of the guide extension catheter 500 disposed within a blood vessel 1300 in a patient's body. As shown, the retractable wire 602 may be extended through the proximal shaft 502 and the secondary lumen 510 so as to function like a buddy wire to assist a guidewire to pass through a challenged lesion area 1302 in the blood vessel 1300. In an embodiment, the blood vessel 1300 may have some "challenged areas" with one or more lesions or obstructions 1302. The retractable wire 602 can pass through the proximal shaft 502 and the distal sheath 506, and thereby facilitate a therapeutic catheter 1306 to cross the challenged areas. In an embodiment, the retractable wire 602 may extend along an exterior of the proximal shaft 502 and extend into the secondary lumen 510 from the proximal opening 524.

As an example, a target portion or area (or the lesion area 1302) within the blood vessel 1300, for example, a coronary artery may be treated. The treatment method may include advancing a guide catheter 1310 through a first outer diameter blood vessel to a location adjacent to an ostium of the coronary artery. The method may also include advancing the guide extension catheter 500 through the guide catheter 1310 to a position where at least a portion of the distal sheath 506 extends beyond a distal end of the guide catheter 1310 and into the coronary artery. Further, a guidewire 1308 may extend within the distal sheath 506 through the guide catheter 1310. Then, the retractable wire 602 may be extended through the proximal shaft 502 and the distal sheath 506 to assist the guidewire 1308 to pass through the challenged lesion area 1302. Similarly, the retractable wire 602 may cross the lesion or the obstruction 1302 by extending distally through the distal end 522. Once the retractable wire 602 passes the challenged lesion area(s) 1302, then the therapeutic catheter 1306 can proceed to a target portion (e.g. 1302) within a vessel that need to be treated. Once the therapeutic catheter 1306 reaches the target portion, such as 1302, the retractable wire 602 can be pulled back outwards or retracted.

The treatment catheter 1306 including a therapeutic device 1304 may be advanced or extended through the guide catheter 1310 and/or the distal sheath 506 of the guide extension catheter 500. The therapeutic device 1304 can hence be delivered to the target portion within the blood vessel 1300 through the guide extension catheter 500. In an embodiment, the therapeutic device 1304 is an angioplasty balloon catheter, a stent delivery system, or the like.

In one aspect, a medical device assembly is disclosed with a guide catheter 1310 (See Figure 13) having a lumen. The medical device may further include a guide extension catheter similar to the guide extension catheter 500 as disclosed in Figures 5 to 13. The guide extension catheter 500 may include the proximal shaft 502 having a first outer diameter 504, and a distal sheath 506 attached to the proximal shaft 502. The distal sheath 506 has a second outer diameter 508 which is greater than the first outer diameter 504 of the proximal shaft 502. Further, the distal sheath 506 has a central lumen 520 defined therein and a secondary lumen 510 formed along a wall surface of the distal sheath 506. In some embodiments, the guide extension catheter 500 may include a guidewire (1308) extending through the central lumen 520. The proximal shaft 502 may be a tubular member 514 with an open distal end 518. The proximal shaft 502 may have a lumen 516 formed therein that is longitudinally-aligned with the secondary lumen 510. The guide extension catheter 500 may have a retractable wire 602 disposed with in at least portion of the proximal shaft 502, the secondary lumen 510 or both. Further, the retractable wire 602 may extend distally from the distal end 522 of the distal sheath 506. The present disclosure also describes a method for accessing a coronary artery or a blood vessel. The method may include providing a guide catheter, and advancing the guide catheter through a first outer diameter blood vessel to a location adjacent to an ostium of a coronary artery. Further, the method may include providing the guide extension catheter and advancing the guide extension catheter through the guide catheter to a position where at least a portion of the distal sheath may extend distally beyond the distal end of the guide catheter and into the coronary artery. Additionally, the method may include advancing a treatment catheter through the guide catheter.

Although the embodiments described above use balloon catheters, those of skill in the art will understand that the principles set out there can be applied to any catheter or endoscopic device where it is deemed advantageous to transmit push force, for example, to a catheter shaft. Conversely, constructional details, including manufacturing techniques and materials, are well within the understanding of those of skill in the art and have not been set out in any detail here. These and other modifications and variations may well within the scope of the present disclosure can be envisioned and implemented by those of skill in the art.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The invention's scope is, of course, defined by the appended claims.

## Claims

1. A guide extension catheter (14; 500), comprising:
a proximal shaft (16; 502) having a first outer diameter (504);
a distal sheath (26; 506) attached to the proximal shaft (16; 502) and having a proximal end (528), a distal end (522), and a second outer diameter (508) greater than the first outer diameter (504); and
wherein the distal sheath (26; 506) has a central lumen (28; 520) defined therein and a secondary lumen (510) formed along a wall surface (512) of the distal sheath (26; 506), the secondary lumen (510) having a proximal opening (524) towards the proximal end (528) of the distal sheath (26; 506) and a distal opening (526) towards the distal end (522) of the distal sheath (26; 506).

2. The guide extension catheter (14; 500) of claim 1, wherein the proximal shaft (16; 502) includes a tubular member (18; 514) with an open distal end (518).

3. The guide extension catheter (14; 500) of any one of claims 1-2,
wherein the proximal shaft (16; 502) has a lumen (22; 516) formed therein that is longitudinally-aligned with the secondary lumen (510).

4. The guide extension catheter (14; 500) of any one of claims 1-3, further comprising a retractable wire (602) disposed within at least a portion of the proximal shaft (16; 502), the secondary lumen (510), or both.

5. The guide extension catheter (14; 500) of claim 4, wherein the secondary lumen (510) is collapsible to shift between an open configuration and a collapsed configuration.

6. The guide extension catheter (14; 500) of claim 5, wherein the secondary lumen (510) is in the open configuration when the retractable wire (602) is disposed therein and wherein the secondary lumen (510) shifts to the collapsed configuration when the retractable wire (602) is retracted therefrom.

7. The guide extension catheter (14; 500) of any one of claims 1-6,
wherein the distal sheath (26; 506) includes a radiopaque material.

8. The guide extension catheter (14; 500) of any one of claims 1-7,
wherein the distal sheath (26; 506) includes a reinforcing member (32; 1003).

9. A medical device assembly, comprising:
a guide catheter (10; 1310) having a lumen (44) formed therein;
a guide extension catheter (14; 500) extending through the lumen (44), the guide extension catheter (14; 500) including:
a proximal shaft (16; 502) having a first outer diameter (504),
a distal sheath (26; 506) attached to the proximal shaft (16; 502) and having a proximal end (528), a distal end (522), and a second outer diameter (508) greater than the first outer diameter (504), and wherein the distal sheath (26; 506) has a central lumen (28; 520) defined therein and a secondary lumen (510) formed along a wall surface (512) of the distal sheath (26; 506), the secondary lumen (510) having a proximal opening (524) towards the proximal end (528) of the distal sheath (26; 506) and a distal opening (526) towards the distal end (522) of the distal sheath (26; 506); and
a guidewire (1308) extending through the central lumen (28; 520) of the distal sheath (26; 506).

10. The medical device assembly of claim 9, wherein the proximal shaft (16; 502) includes a tubular member (18; 514) with an open distal end (518).

11. The medical device assembly of any one of claims 9-10, wherein the proximal shaft (16; 502) has a lumen (22; 516) formed therein that is longitudinally-aligned with the secondary lumen (510).

12. The medical device assembly of any one of claims 9-10, wherein a retractable wire (602) is disposed within the proximal shaft (16; 502).

13. The medical device assembly of claim 12, wherein the retractable wire (602) is disposed within the secondary lumen (510).

14. The medical device assembly of claim 13, wherein the retractable wire (602) extends distally from a distal end (522) of the distal sheath (26; 506).

15. The medical device assembly of any one of claims 9-14, wherein the secondary lumen (510) is configured to shift between an open configuration and a collapsed configuration and wherein the secondary lumen (510) is in the open configuration when a retractable wire (602) is disposed therein and wherein the secondary lumen (510) shifts to the collapsed configuration when the retractable wire (602) is retracted therefrom.

## Patentansprüche

1. Führungsverlängerungskatheter (14; 500), der aufweist:
einen proximalen Schaft (16; 502) mit einem ersten Außendurchmesser (504);
eine distale Hülle (26; 506), die am proximalen Schaft (16; 502) befestigt ist und ein proximales Ende (528), ein distales Ende (522) und einen zweiten Außendurchmesser (508) hat, der größer als der erste Außendurchmesser (504) ist; und
wobei die distale Hülle (26; 506) ein darin definiertes Zentrallumen (28; 520) und ein Sekundärlumen (510) hat, das entlang einer Wandfläche (512) der distalen Hülle (26; 506) gebildet ist, wobei das Sekundärlumen (510) eine proximale Öffnung (524) zum proximalen Ende (528) der distalen Hülle (26; 506) und eine distale Öffnung (526) zum distalen Ende (522) der distalen Hülle (26; 506) hat.

2. Führungsverlängerungskatheter (14; 500) nach Anspruch 1, wobei der proximale Schaft (16; 502) ein Röhrenteil (18; 514) mit einem offenen distalen Ende (518) aufweist.

3. Führungsverlängerungskatheter (14; 500) nach Anspruch 1 oder 2, wobei der proximale Schaft (16; 502) ein darin gebildetes Lumen (22; 516) hat, das zum Sekundärlumen (510) längs-ausgerichtet ist.

4. Führungsverlängerungskatheter (14; 500) nach einem der Ansprüche 1 bis 3, ferner mit einem zurückziehbaren Draht (602), der in mindestens einem Abschnitt des proximalen Schafts (16; 502) und/oder des Sekundärlumens (510) angeordnet ist.

5. Führungsverlängerungskatheter (14; 500) nach Anspruch 4, wobei das Sekundärlumen (510) zusammenlegbar ist, um zwischen einer offenen Konfiguration und einer zusammengelegten Konfiguration überzugehen.

6. Führungsverlängerungskatheter (14; 500) nach Anspruch 5, wobei sich das Sekundärlumen (510) in der offenen Konfiguration befindet, wenn der zurückziehbare Draht (602) darin angeordnet ist und wobei das Sekundärlumen (510) in die zusammengelegte Konfiguration übergeht, wenn der zurückziehbare Draht (602) daraus zurückgezogen ist.

7. Führungsverlängerungskatheter (14; 500) nach einem der Ansprüche 1 bis 6, wobei die distale Hülle (26; 506) ein röntgendichtes Material aufweist.

8. Führungsverlängerungskatheter (14; 500) nach einem der Ansprüche 1 bis 7, wobei die distale Hülle (26; 506) ein Verstärkungsteil (32; 1003) aufweist.

9. Medizinproduktanordnung, die aufweist:
einen Führungskatheter (10; 1310) mit einem darin gebildeten Lumen (44);
einen Führungsverlängerungskatheter (14; 500), der sich durch das Lumen (44) erstreckt, wobei der Führungsverlängerungskatheter (14; 500) aufweist:
einen proximalen Schaft (16; 502) mit einem ersten Außendurchmesser (504) ;
eine distale Hülle (26; 506), die am proximalen Schaft (16; 502) befestigt ist und ein proximales Ende (528), ein distales Ende (522) und einen zweiten Außendurchmesser (508) hat, der größer als der erste Außendurchmesser (504) ist, und wobei die distale Hülle (26; 506) ein darin definiertes Zentrallumen (28; 520) und ein Sekundärlumen (510) hat, das entlang einer Wandfläche (512) der distalen Hülle (26; 506) gebildet ist, wobei das Sekundärlumen (510) eine proximale Öffnung (524) zum proximalen Ende (528) der distalen Hülle (26; 506) und eine distale Öffnung (526) zum distalen Ende (522) der distalen Hülle (26; 506) hat; und
einen Führungsdraht (1308), der sich durch das Zentrallumen (28; 520) der distalen Hülle (26; 506) erstreckt.

10. Medizinproduktanordnung nach Anspruch 9, wobei der proximale Schaft (16; 502) ein Röhrenteil (18; 514) mit einem offenen distalen Ende (518) aufweist.

11. Medizinproduktanordnung nach Anspruch 9 oder 10, wobei der proximale Schaft (16; 502) ein darin gebildetes Lumen (22; 516) hat, das zum Sekundärlumen (510) längs-ausgerichtet ist.

12. Medizinproduktanordnung nach Anspruch 9 oder 10, wobei ein zurückziehbarer Draht (602) im proximalen Schaft (16; 502) angeordnet ist.

13. Medizinproduktanordnung nach Anspruch 12, wobei der zurückziehbare Draht (602) im Sekundärlumen (510) angeordnet ist.

14. Medizinproduktanordnung nach Anspruch 13, wobei sich der zurückziehbare Draht (602) von einem distalen Ende (522) der distalen Hülle (26; 506) distal erstreckt.

15. Medizinproduktanordnung nach einem der Ansprüche 9 bis 14, wobei das Sekundärlumen (510) dafür konfiguriert ist, zwischen einer offenen Konfiguration und einer zusammengelegten Konfiguration überzugehen und wobei sich das Sekundärlumen (510) in der offenen Konfiguration befindet, wenn ein zurückziehbarer Draht (602) darin angeordnet ist und wobei das Sekundärlumen (510) in die zusammengelegte Konfiguration übergeht, wenn der zurückziehbare Draht (602) daraus zurückgezogen ist.

## Revendications

1. Cathéter d'extension de guidage (14 ; 500) comprenant :
un arbre proximal (16 ; 502) ayant un premier diamètre externe (504) ;
une gaine distale (26 ; 506) fixée à l'arbre proximal (16 ; 502) et ayant une extrémité proximale (528), une extrémité distale (522) et un second diamètre externe (508) supérieur au premier diamètre externe (504) ; et
dans lequel la gaine distale (26 ; 506) a une lumière centrale (28 ; 520) définie à l'intérieur de cette dernière et une lumière secondaire (510) formée le long d'une surface de paroi (512) de la gaine distale (26 ; 506), la lumière secondaire (510) ayant une ouverture proximale (524) vers l'extrémité proximale (528) de la gaine distale (26 ; 506) et une ouverture distale (526) vers l'extrémité distale (522) de la gaine distale (26 ; 506).

2. Cathéter d'extension de guidage (14 ; 500) selon la revendication 1, dans lequel l'arbre proximal (16 ; 502) comprend un élément tubulaire (18 ; 514) avec une extrémité distale (518) ouverte.

3. Cathéter d'extension de guidage (14 ; 500) selon l'une quelconque des revendications 1 à 2, dans lequel l'arbre proximal (16 ; 502) a une lumière (22 ; 516) formée à l'intérieur de ce dernier, qui est alignée longitudinalement avec la lumière secondaire (510).

4. Cathéter d'extension de guidage (14 ; 500) selon l'une quelconque des revendications 1 à 3, comprenant en outre un fil rétractable (602) disposé à l'intérieur d'au moins une partie de l'arbre proximal (16 ; 502), la lumière secondaire (510) ou les deux.

5. Cathéter d'extension de guidage (14 ; 500) selon la revendication 4, dans lequel la lumière secondaire (510) est repliable pour se déplacer entre une configuration ouverte et une configuration repliée.

6. Cathéter d'extension de guidage (14 ; 500) selon la revendication 5, dans lequel la lumière secondaire (510) est dans la configuration ouverte lorsque le fil rétractable (602) est disposé à l'intérieur de ce dernier, et dans lequel la lumière secondaire (510) passe dans la configuration repliée lorsque le fil rétractable (602) est rétracté à partir de cette dernière.

7. Cathéter d'extension de guidage (14 ; 500) selon l'une quelconque des revendications 1 à 6, dans lequel la gaine distale (26 ; 506) comprend un matériau radio-opaque.

8. Cathéter d'extension de guidage (14 ; 500) selon l'une quelconque des revendications 1 à 7, dans lequel la gaine distale (26 ; 506) comprend un élément de renforcement (32 ; 1003).

9. Ensemble de dispositif médical comprenant :
un cathéter de guidage (10 ; 1310) ayant une lumière (44) formée à l'intérieur de ce dernier ;
un cathéter d'extension de guidage (14 ; 500) s'étendant à travers la lumière (44), le cathéter d'extension de guidage (14 ; 500) comprenant :
un arbre proximal (16 ; 502) ayant un premier diamètre externe (504),
une gaine distale (26 ; 506) fixée à l'arbre proximal (16 ; 502) et ayant une extrémité proximale (528), une extrémité distale (522) et un second diamètre externe (508) supérieur au premier diamètre externe (504), et dans lequel la gaine distale (26 ; 506) a une lumière centrale (28 ; 520) définie à l'intérieur de cette dernière et une lumière secondaire (510) formée le long d'une surface de paroi (512) de la gaine distale (26 ; 506), la lumière secondaire (510) ayant une ouverture proximale (524) vers l'extrémité proximale (528) de la gaine distale (26 ; 506) et une ouverture distale (526) vers l'extrémité distale (522) de la gaine distale (26 ; 506) ; et
un fil-guide (1308) s'étendant à travers la lumière centrale (28 ; 520) de la gaine distale (26 ; 506).

10. Ensemble de dispositif médical selon la revendication 9, dans lequel l'arbre proximal (16 ; 502) comprend un élément tubulaire (18 ; 514) avec une extrémité distale ouverte (518).

11. Ensemble de dispositif médical selon l'une quelconque des revendications 9 à 10, dans lequel l'arbre proximal (16 ; 502) a une lumière (22 ; 516) formée à l'intérieur de ce dernier qui est alignée longitudinalement avec la lumière secondaire (510).

12. Ensemble de dispositif médical selon l'une quelconque des revendications 9 à 10, dans lequel un fil rétractable (602) est disposé à l'intérieur de l'arbre proximal (16 ; 502).

13. Ensemble de dispositif médical selon la revendication 12, dans lequel le fil rétractable (602) est disposé à l'intérieur de la lumière secondaire (510).

14. Ensemble de dispositif médical selon la revendication 13, dans lequel le fil rétractable (602) s'étend de manière distale à partir d'une extrémité distale (522) de la gaine distale (26 ; 506).

15. Ensemble de dispositif médical selon l'une quelconque des revendications 9 à 14, dans lequel la lumière secondaire (510) est configurée pour se déplacer entre une configuration ouverte et une configuration repliée et dans lequel la lumière secondaire (510) est dans la configuration ouverte lorsque le fil rétractable (602) est disposé à l'intérieur de cette dernière et dans lequel la lumière secondaire (510) passe dans la configuration repliée lorsque le fil rétractable (602) est rétracté à partir de cette dernière.
